# EUROPEAN PATENT SPECIFICATION

(11) **EP 2 370 137 B1**
(45) Date of publication and mention of the grant of the patent: **11.03.2020**
(21) Application number: 09748695.5
(22) Date of filing: 10.11.2009
(51) Int. Cl.: A61M 25/00

(54) **COUPLING ARRANGEMENT FOR A TELESCOPIC DEVICE**
KOPPLUNGSANORDNUNG FÜR EIN TELESKOPGERÄT
AGENCEMENT DE COUPLAGE POUR UN DISPOSITIF TÉLESCOPIQUE

(30) Priority: 14.11.2008 DK 200801587
(43) Date of publication of application: 05.10.2011
(73) Proprietor: Coloplast A/S, 3050 Humlebæk (DK)
(72) Inventor: TORSTENSEN, Jan, DK-2830 Virum (DK)
(86) International application number: PCT/DK2009/050295
(87) International publication number: WO 2010/054659

(56) References cited:
- EP-A1- 0 935 974
- WO-A-2005/056099
- WO-A-2006/045809
- WO-A-2008/138352
- US-A- 5 846 259
- US-A1- 2003 105 451

## Description

### TECHNOLOGY FIELD

The present invention relates to a telescopic device and a coupling arrangement for coupling the telescopic device in an extended configuration. In particular the invention relates to a telescopic catheter and a coupling arrangement for coupling the telescopic device in a ready-to-use configuration.

### BACKGROUND

The use of intermittent catheters has become almost a standard for persons not able to urinate of free will. Such users, typically paralysed persons such as para- and tetraplectics, have found that using intermittent catheters has greatly improved their freedom to move around and lead an active life as catheterisation can be performed anywhere.

However, in order for the user to come out publicly and socialise it has become more and more important that such products are discreet and easy to carry around. Thus, a demand for compact catheters, which can easily be stored and carried around in handbags or pockets, has grown.

In order to fulfil such needs, products such as the SpeediCath® Compact, produced by Coloplast A/S have been developed. However, this product mainly targets female users. Male users have a much longer urinary channel and thus other demands and requirements are to be fulfilled for a male product.

Many of these issues and solutions thereto have been discussed in WO 2006/045809, which discloses an expandable catheter with a transition between the individual sections allowing insertion of the transition into urethra. Document WO 2006/045809 discloses all the features of the preamble of claim 1.

However, there is still a need for alternative and improved solutions as will be discussed herein.

### SUMMARY OF THE INVENTION

The present disclosure relates to a telescopic intermittent urinary catheter, comprising a first tubular element and an extension member displaceably arranged in an axial direction within the first tubular element, between at least, a first axial position wherein the extension member is displaceable within the first tubular element, where the first tubular element comprises a first coupling means and the extension member comprises a second coupling means, and in a second axial position where the first and second coupling means cooperate with each other for limiting the displacement of said extension member in at least one longitudinal direction, where the first coupling and/or the second coupling means are provided with at least one protrusion in a radial direction of a longitudinal axis of the first tubular element and/or the extension member.

The telescopic device of the present invention is an intermittent urinary catheter which may be used to drain a human urinary bladder of urine. The telescopic device is at least constructed from two elements, the first tubular element and the extension member, where the extension member is arranged as being moveable in a direction defined by the longitudinal axis of the extension member or the first tubular element. During storage, transport or other non-use activities of the telescopic device, the device may be kept in a non-extended position, where the length of the device is minimized.

In order to allow a user of the intermittent catheter, to use it in its extended position, the extension member is manoeuvred such that the first coupling means of the first tubular element, and the second coupling means of the extension member come in contact with each other and engage with each other such that the extension member is maintained in its extended position. Thereby, the user may introduce the telescopic device into the urinary channel and into the bladder and drain the urine from the bladder.

When the telescopic device is in its second axial position, such as the extended position, the first and/or the second coupling means are arranged in such a way that at least parts of the protrusions are in contact with each other such that the interaction between the protrusions prevents the telescopic device from being manoeuvred into its collapsed or first axial position.

Within the context of the present invention the term radial direction is meant as something which is made in the direction of a radius, going from a centre outwards or from the circumference and inwards.

In one embodiment of the present invention the at least one protrusion of the first coupling means may extend at least one revolution of the inner surface of the first tubular element. This means that when the first tubular element is viewed along its longitudinal axis, the inner diameter is decreased in the area where the protrusion is provided.

The above mentioned protrusions may be used to increase the external diameter of a tubular element in a specific area of the element such as the extension member and/or to decrease the internal diameter of a tubular element in a specific area, such as the first tubular element. This means that at least some part of the protrusion of the second coupling means will be in cooperation with the protrusions of the first coupling means when the telescopic element is in its second axial position.

According to the present invention, the first coupling means are arranged on the internal surface of the first tubular element.

It should be understood within the meaning of the present invention that the term internal can mean any part of the first tubular element, which cannot be construed as facing the exterior of the first tubular element. This may include the inner surface of the tubular element, any form of groove or extrusion on the inside of the tubular element or any surface area that is not facing the exterior or the outside of the tubular element, e.g. facing inwards and towards the central longitudinal axis of the first tubular element or the extension member.

By arranging the first coupling means on the internal surface of the first tubular element the outer surface of the first tubular element, which may be intended to be inserted into the urinary channel, may be kept smooth and without any depression, protrusions or deformities in order to minimize the risk of damaging or injuring the sensitive mucous tissue of the urinary channel.

Comparatively, in one embodiment of the present invention the second coupling means may be arranged on the outer surface of the extension member, such that the outer surface of the extension member engages the internal surface of the first tubular element. This means that neither of the coupling means are accessible from the outside of the telescopic device in the situation where the telescopic device has been arranged in its extended position, as the first and/or the second coupling means are both within the interior first tubular element.

In order to ensure that the telescopic intermittent urinary catheter may be extended to a length, that may be defined as close to the length of the first tubular element and the length of the extension member added together, the first coupling means may be provided at the proximal end of the first tubular element and the second coupling mean may be provided at the distal end of the extension member.

Within the meaning of the present invention the term proximal means a part which is situated nearer to the point of reference, such as the human body during insertion of the telescopic device into a urinary channel of a person. Similarly, the term distal means a part which is situated far from the point of reference, such as the human body as mentioned above. By arranging the first coupling means and the second coupling means at the proximal end of the first tubular element and the distal end of the extension member, respectively, the telescopic device may be extended to a length which is close to the length of the first tubular element plus the length of the extension member. As an example, if the first tubular element is 10 cm long and the extension member is 10 cm long, the telescopic device is approximately 10 cm long in its shortened state, while it may be close to 20 cm long in its extended state. It may be appreciated that any variation in length of either the first tubular element or the extension member is within the capabilities of the skilled person based on the present disclosure.

In the present invention the first coupling means are provided as a helical protrusion arranged on the internal surface of the first tubular element. The helical protrusion decreases the internal diameter of the first tubular element such that the protrusion may be used as a stopping means for the second coupling means of the extension member and preventing the extension member from sliding into the first tubular element after it has been arranged in its extended position. This means that any force is asserted in onto the proximal end of the extension member in a longitudinal direction of the telescopic device, the extension member will be susceptible to maintain its extended position.

The helical or non-helical protrusion on the internal surface of the first tubular element may be arranged such that the protrusion may be relatively rigid. This means that when the extension member is manoeuvred into its extended position the protrusion may not resiliently deform or permanently deform, as any deformation of the protrusion in a radial or longitudinal direction may be transferred into the material of the first tubular element and deform it.

In order to minimize the risk that the first tubular element may be deformed during the manoeuvring of the extension member into its extended position, the second coupling means may comprise a protrusion extending in an radial direction which in its unloaded configuration may have a first diameter that may be greater that the diameter of extension member.

Within the context of the present invention the term unloaded means when there is little or no external force applied to an element, such as the protrusion of the first or second coupling means. The external force is the force which may be defined as force applied in a radial direction, i.e. towards or away from a longitudinal axis. The term loaded means when there is an application of an external force to an element, where the external force is defined as above.

This means that the part of the extension member that is not provided with a protrusion in a radial direction may not apply any force on the first coupling means during the manoeuvring of the extension member into its extended position. However, when the second coupling means interacts with the first coupling means, any force in a radial direction may be exerted by the protrusion of the second coupling means. Thus, the second coupling means may provide the force necessary to prevent the extension member to slide into the first tubular element and maintain its extended state during the insertion of the telescopic device.

Also described is that the second coupling means may comprise a protrusion extending in a radial direction which in its loaded configuration may have a second diameter that may be smaller than the first diameter of the second coupling means. This means that when any radial force is applied to the protrusion of the second coupling means, the diameter of the protrusion may be decreased from a first diameter to a smaller second diameter. Thus, when the second coupling means encounter a constriction in the first tubular element, such as the first coupling means, the diameter of the second coupling means will decrease and may pass the constriction more easily than if the second coupling means had a rigid protrusion that would maintain its diameter in its loaded and unloaded configuration.

After the second coupling means have cleared the constriction in the first tubular element, and there is little or no external force applied to the protrusion of the second coupling means the diameter of the protrusion of the second coupling means may return from its second diameter to its first diameter. This means that the second coupling means may prevent the extension member to be manoeuvred from its extended state during the insertion of the telescopic device into a body orifice, such as the urinary channel.

The said protrusion in a radial direction of the second coupling means may be resiliently arranged between a first diameter in an unloaded state and a second diameter in a loaded state that is smaller than the first diameter. By arranging the protrusion resiliently between a first and a second diameter, the protrusion will return to its original first diameter when there is no radial force applied to the protrusion or the second coupling member.

The present disclosure also relates to a method of manufacturing a telescopic intermittent urinary catheter as defined above, comprising the steps of, providing an extension member having at least an insertable catheter tip, a catheter body having an internal conduit and a second coupling means, injecting a fluid catheter material into a mould formed to define a first tubular element having an internal conduit of a size allowing the extension member to be displaceably positioned inside the first tubular element, where the first tubular element is provided with a moulded first coupling means to cooperate with the second coupling means of the extension member, and solidifying the material therein.

The mould for the first tubular element may be an oblong hollow, tubular part which has an internal shape and structure to define the outer shape and structure of the first tubular element. After the moulding material has been solidified, the extension member may be inserted into the internal conduit where the extension member may be provided as the telescopic part by extending it out of the first tubular element.

In one embodiment, the first tubular element may be moulded around a moulding member defining the internal conduit of the first tubular element and the first coupling means. This means that in addition to the external surface being moulded into shape using the internal shape of the mould, the internal surface or inside of the first tubular element and the first coupling means are shaped by the moulding member. Thus, the shape, structure and size of the first tubular element may be defined by the mould and the moulding member.

In the invention, the first coupling means are moulded to define a helical protrusion in a radial direction on the internal surface of the first tubular element. The shape, structure and size of the helical protrusion may usually be defined using the internal moulding member while it may be obvious to the skilled person that the protrusions may be achieved using modifications of the present method.

In another embodiment of the present invention, the first tubular element may be removed from the mould without deforming the moulded material during removal. This may be achieved by moulding the first tubular element in such a way that neither the mould or the moulding element leave impressions or structures in the moulded material, that require the moulded material to be deformed by stretching, compressing or bending when removing the first tubular element from the mould or from the moulding element.

In one embodiment of the present invention, the first tubular element may be removed from the mould by manoeuvring the first tubular element in a circular movement around the longitudinal axis of the first tubular element releasing the moulding member from the first coupling means. This is especially advantageous when the first coupling means are provided as a helical structure or thread. Thus, the circular movement allows the first tubular element to be unscrewed off the moulding member, similar to unscrewing a nut from a stationary bolt.

In one embodiment of the present invention, the first tubular element may be removed from the mould by manoeuvring the moulding member in a circular movement around its longitudinal axis releasing the moulding member from the first coupling means. This is especially advantageous when the first coupling means are provided as a helical structure or thread. Thus, the circular movement of the moulding member allows the moulding member to be unscrewed from the first tubular member, similar to unscrewing a bolt from a stationary nut.

### BRIEF DESCRIPTION OF THE DRAWINGS

The invention will be discussed further with reference to the following example embodiments, wherein
Fig. 1 is a sectional side view of a telescopic device - where the telescopic device is in its first axial position,
Fig. 2 is a sectional side view of the same, where the telescopic device is in its second axial position,
Fig. 3 is a sectional side view of a proximal end of the first tubular member, and
Fig. 4 is a sectional side view of the proximal end of the first tubular member and a distal end of an extension member of a telescopic device in its second axial position.

### DETAILED DESCRIPTION

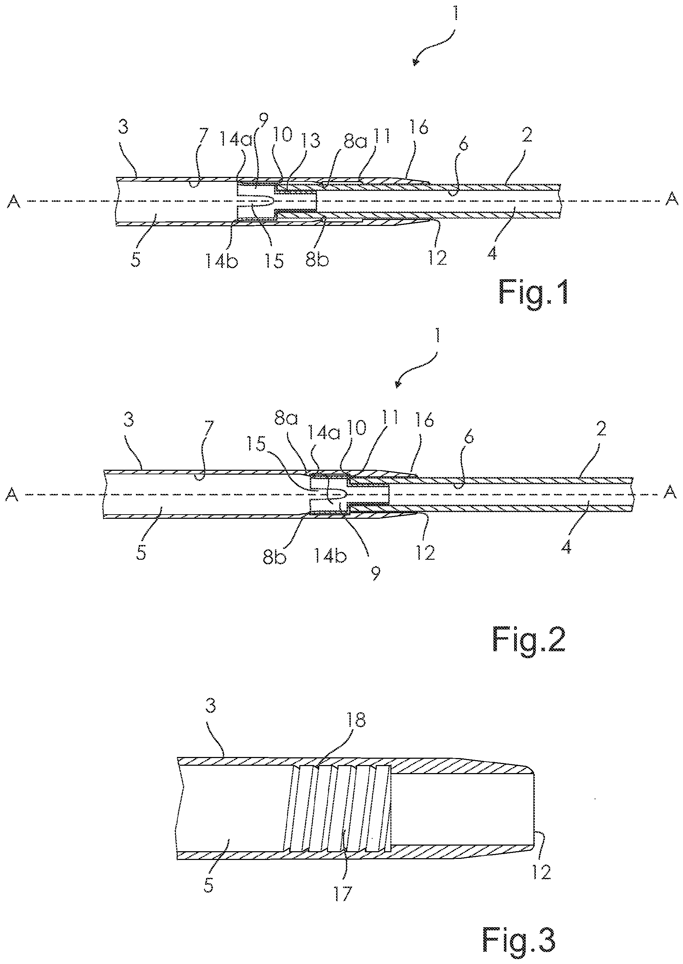

Fig. 1 shows a sectional diagram of a telescopic urinary catheter 1 which has an extension member 2 and a first tubular member 3, where the extension member 2 is displaceably arranged within the first tubular member 3. This means that the extension member 2 may be manoeuvred freely in a direction along the longitudinal axis A. The extension member 2, which is inserted into the urinary bladder of a patient, has an internal lumen 4 which allows urine to flow from the bladder and out of the telescopic catheter via the internal lumen 5 of the first tubular member 3. During the usage of the urinary catheter 1 the urine flows along a path within the catheter 1 which is parallel to the longitudinal axis A.

The first tubular member 3 is provided with a first coupling means 8a, 8b on the internal surface 7of the first tubular member 3. The first coupling means 8a, 8b are formed as protrusions in a radial direction from the internal surface 7 and towards the longitudinal axis A. The protrusions 8a, 8b may be formed to be continuous as a closed circle along the internal surface 7 of the first tubular member, where the protrusions reduce the diameter of the inner lumen 5 of the first tubular member 3 in a specific area.

The extension member 2 may be provided with a coupling member 9 which has at least two separate functions. The first function is to prevent the extension member 2 to slide out of the first tubular member 2, when the extension member 3 is manoeuvred into the catheter's 1 extended state. This may be achieved using a proximal ridge 10 on the coupling member which cooperates with a distal ridge 11 on the first tubular member 3, such that the diameter of the proximal ridge 10 is larger than the distal ridge 11 and thus prevents the extension member 3 from exiting the proximal end 12 of the first tubular member 3.

The second function of the coupling member 9 may be to prevent the extension member 2 from sliding back into the first tubular member 3 after the extension member 2 has been extended into the catheter's 1 extended position. The coupling member may be provided with a second coupling means 14a, 14b which may be provided as protrusions in a radial direction from the longitudinal axis and outwards. The second coupling means 14a,14b may have a first diameter that is of similar to or the same size as the inner diameter of the first tubular member 3 and when the second coupling means 14a,14b are manoeuvred past the first coupling means 8a,8b during the extension of the catheter 1 the diameter of the second coupling means 14a,14b may, under the radial force exerted from the first coupling means 8a,8b, be reduced to a second diameter that is smaller than the inner diameter of the first coupling means 8a,8b such that the force exerted in the longitudinal direction to extend the catheter may be minimized.

The transition from the first diameter of the second coupling means 14a, 14b to a second diameter that is smaller than the first diameter, may be achieved by having the second coupling means 14a, 14b resiliently move from the first diameter to the second diameter. The resiliency may be provided by arranging cut-outs 15 in the sides of the coupling member, which increase the compressability of the distal ends of the coupling means. It is to be understood that the choice of material, the dimensions and the build of the coupling member has an influence of the compressability of the distal ends, and the appropriate choice of material is within the capabilities of the skilled person.

The coupling member 9 may be an integral part of the extension member, such as by moulding the extension member 2 and the coupling member 9 together or as in this embodiment where the coupling member 9 is provided as a separate part which has a neck area 13 which is inserted into the internal lumen 4 of the extension member 2 and permanently attached to the inner surface 6 of the extension member.

The first coupling means 8 and the second coupling means 14 may be tapered on one side and have a sharp edge on the opposite side, such that when the first coupling means 8 come in contact with the second coupling means, the tapered surfaces are in contact with each other. As the coupling member is in its locked position, the sharp edges are in contact with each other, preventing the movement of the coupling member in a distal direction along the longitudinal axis A. The distal end 12 of the first tubular member 3 may be tapered 16 in order to ease the transition from the external diameter of the extension member 2 and the external diameter of the first tubular member 3, such that there are no sharp edges which may harm or injure the walls of the urinary channel during insertion of the catheter 1.

Fig. 2 shows the catheter 1 in its extended position, where the proximal ridge 10 of the coupling member 9 is in contact with the distal ridge 11 of the first tubular member 3, which prevents the extension member to be extended out of the proximal end 12 of the first tubular member 3. The second coupling means 14a, 14b are located proximally to the first coupling means 8 and the first coupling means 8a, 8b prevent the extension member 2 to be manoeuvred in a distal direction along the longitudinal axis A. This means that the extension member 2 is locked in its extended position, and the catheter 1 may be inserted into the urinary channel of a user without risking the collapse of the telescopic catheter 1.

The extension member 2 and/or the first tubular member may be produced of rather soft materials such as polyurethane, PVC (polyvinylchloride) or similar flexible materials and the distal section may for example be produced of hard materials such as polyurethane, polyolefines, PEEK (polyetheretherketon), PC (polycarbonate), PET (polyester, polyethylenephtalate), ABS (acrylonitril-butadien-styrene) and/or MABS (methylmethacrylate acrylonitril-butadien-styrene). As can be seen some materials, for example polyurethane, can be used for both the first tubular element 3 and the extension member 2, although with different hardness.

The coupling member 9 is typically formed of a relatively stiff material in order to prevent deformation of the proximal part other than the intended resilient movement obtained by the cut-outs 15. Such materials can be numerous and selected between many different plastics but also aluminium, steel, brass etc. In order to be able to attach or weld the coupling member 9 to the extension member 2 a polyurethane may be used, for example Desmopan as mentioned above. Other plastic materials can for example be polyolefins, such as polypropylene, polyethylene, EVA (polyethylene vinylacetate copolymer), ABS MABS, Kraton, PET, PC, PCTG(copolyester/polycarbonat) blends, HIPS (high impact polystyrene), PA (polyamid), SAN (styrene-acrylonitril), PS (polystyrene) and SEBS (styrene-ethylene/bothylene-styrene).

Fig. 3 shows a sectional view of a proximal end 12 of a first tubular member 3 according to the invention, where the second coupling means are in the form of a helical structure 17. The helical structure is in principle a single radial protrusion 18 which wounds helically around the inner surface 6 of the first tubular member, similarly to the inner threads of a nut. The number of full revolutions of the protrusion may be from a single revolution and up to five revolutions, depending on the preferences of the producer and/or designer of the catheter. By having the second coupling means as a helical structure it is easy to mould the first tubular element having an moulding member on the inside of the first tubular element, where the moulding member may be removed from the moulded material by screwing the member off the first tubular element.

The helical structure 17 of the second coupling means may be adjusted to the preference of the manufacturer or the producer, such that the form, shape and size of the helical structure 17 meets the requirements for maintaining the extension member 2 in its extended position. Furthermore, the moulding member used to shape the helical thread is provided with depressions that, when filled, provide the helical protrusions in the first tubular member 3. The depressions in the moulding member may be continuous with along the helical structure, and may be open ended such that when the moulding member and the first tubular member are rotated to release the moulding member or the tubular member, there will be no hindrance in the first tubular member or the moulding member and the helical structure will follow the depressions until the helical structure releases the depressions of the moulding member.

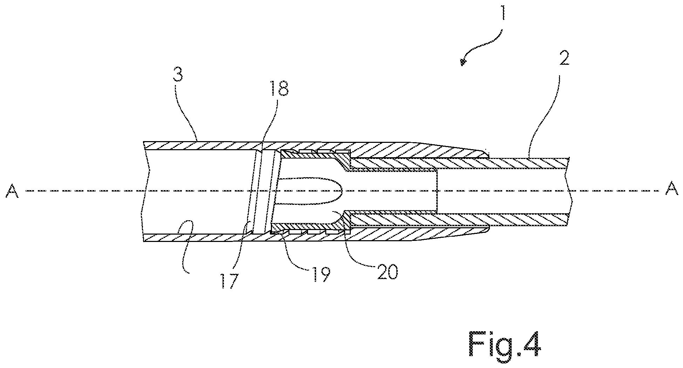

Fig. 4 shows a sectional view of the central area of the urinary catheter 1, having the first tubular member of Fig. 3 where the second coupling means 17 are a helical structure according to the invention. The first coupling means 19 of the coupling member 20 are tilted such that the first coupling means 19 follow the contour of the second coupling means 17 at least one revolution. The first coupling means 19 may be screwed into the second coupling means 17.

## Claims

1. A telescopic intermittent urinary catheter (1) comprising a first tubular element (3) and an extension member (2) displaceably arranged in an axial direction within the first tubular element (3), between at least,
- a first axial position wherein the extension member (2) is displaceable within the first tubular element (3), where the first tubular element (3) comprises a first coupling means (17) and the extension member (2) comprises a second coupling means (19), and
- in a second axial position where the first and second coupling means (17, 19) cooperate with each other for limiting the displacement of said extension member (2) in at least one longitudinal direction
- where the first and/or the second coupling means (17, 19) are provided with at least one protrusion in a radial direction of a longitudinal axis of the first tubular element and/or the extension member
- **characterised in that** the first coupling means (17) is provided as a helical protrusion arranged on the internal surface of the tubular element.

2. A telescopic intermittent urinary catheter (1) according to any of the preceding claims, wherein the second coupling means (19) is arranged on the outer surface of the extension member.

3. A telescopic intermittent urinary catheter (1) according to any of the preceding claims, wherein the first coupling means (17) is provided at the proximal end of the first tubular element and the second coupling (19) mean is provided at the distal end of the extension member.

4. A method of manufacturing a telescopic intermittent urinary catheter (1) according to claims 1 to 3, comprising the steps of
- providing an extension member (2) having at least an insertable catheter tip, a catheter body having an internal conduit and a second coupling means (19),
- injecting a fluid catheter material into a mould formed to define a first tubular element (3) having an internal conduit of a size allowing the extension member (2) to be displaceably positioned inside the first tubular element (3),
- where the first tubular element (3) is provided with a moulded first coupling means (17) to cooperate with the second coupling means (19) of the extension member, and
- solidifying the material therein
- wherein the first coupling means are moulded to define a helical protrusion (17) in a radial direction on the internal surface of the first tubular element.

5. A method according to claim 4, wherein the first tubular element (3) is moulded around a moulding member defining the internal conduit of the first tubular element and the first coupling means (17).

6. A method according to any of the claims 4-5, wherein the first tubular element (3) is removed from the mould without deforming the moulded material during removal.

7. A method according to any one of the claims 4-6, wherein the first tubular element (3) is removed from the mould by manoeuvring the first tubular element (3) in a circular movement around the longitudinal axis of the first tubular element (3) releasing the moulding member from the first coupling means (17).

8. A method according to any one of the claims 4-7, wherein the first tubular element (3) is removed from the mould by manoeuvring the moulding member in a circular movement around its longitudinal axis releasing the moulding member from the first coupling means (17).

## Patentansprüche

1. Teleskopischer intermittierender Blasenkatheter (1), umfassend ein erstes röhrenförmiges Element (3) und ein Verlängerungselement (2), das in einer axialen Richtung in dem ersten röhrenförmigen Element (3) verschiebbar angeordnet ist zwischen wenigstens:
- einer ersten axialen Position, wobei das Verlängerungselement (2) in dem ersten röhrenförmigen Element (3) verschiebbar ist, wobei das erste röhrenförmige Element (3) ein erstes Kopplungsmittel (17) umfasst und das Verlängerungselement (2) ein zweites Kopplungsmittel (19) umfasst, und
- einer zweiten axialen Position, wobei das erste und das zweite Kopplungsmittel (17, 19) zusammenwirken, um die Verschiebung des Verlängerungselements (2) in wenigstens einer Längsrichtung zu begrenzen,
- wobei das erste und/oder das zweite Kopplungsmittel (17, 19) mit wenigstens einem Vorsprung in einer radialen Richtung einer Längsachse des ersten röhrenförmigen Elements und/oder des Verlängerungselements versehen sind bzw. ist,
- **dadurch gekennzeichnet, dass** das erste Kopplungsmittel (17) als ein spiralförmiger Vorsprung vorgesehen ist, der auf der Innenfläche des röhrenförmigen Elements angeordnet ist.

2. Teleskopischer intermittierender Blasenkatheter (1) nach einem der vorhergehenden Ansprüche, wobei das zweite Kopplungsmittel (19) auf der Außenfläche des Verlängerungselements angeordnet ist.

3. Teleskopischer intermittierender Blasenkatheter (1) nach einem der vorhergehenden Ansprüche, wobei das erste Kopplungsmittel (17) an dem nahe gelegenen Ende des ersten röhrenförmigen Elements vorgesehen ist und das zweite Kopplungsmittel (19) an dem entfernt gelegenen Ende des Verlängerungselements vorgesehen ist.

4. Verfahren zur Herstellung eines teleskopischen intermittierenden Blasenkatheters (1) nach den Ansprüchen 1 bis 3, umfassend die folgenden Schritte:
- Bereitstellen eines Verlängerungselements (2), das wenigstens eine einsetzbare Katheterspitze, einen Katheterkörper mit einem Innenkanal und ein zweites Kopplungsmittel (19) aufweist,
- Einspritzen eines flüssigen Kathetermaterials in eine Form, die so ausgebildet ist, dass sie ein erstes röhrenförmiges Element (3) definiert, das einen Innenkanal mit einer Größe aufweist, die ermöglicht, dass das Verlängerungselement (2) verschiebbar in dem ersten röhrenförmigen Element (3) positioniert ist,
- wobei das erste röhrenförmige Element (3) mit einem ausgeformten ersten Kopplungsmittel (17) versehen ist, das mit dem zweiten Kopplungsmittel (19) des Verlängerungselements zusammenwirken soll, und
- Verfestigen des Materials in demselben,
- wobei das erste Kopplungsmittel so ausgeformt ist, dass es einen spiralförmigen Vorsprung (17) in einer radialen Richtung auf der Innenfläche des ersten röhrenförmigen Elements definiert.

5. Verfahren nach Anspruch 4, wobei das erste röhrenförmige Element (3) um ein Formelement herum ausgeformt ist, das den Innenkanal des ersten röhrenförmigen Elements und das erste Kopplungsmittel (17) definiert.

6. Verfahren nach einem der Ansprüche 4 bis 5, wobei das erste röhrenförmige Element (3) aus der Form entnommen wird, ohne dass das ausgeformte Material während des Entnehmens verformt wird.

7. Verfahren nach einem der Ansprüche 4 bis 6, wobei das erste röhrenförmige Element (3) aus der Form entnommen wird, indem das erste röhrenförmige Element (3) in einer kreisförmigen Bewegung um die Längsachse des ersten röhrenförmigen Elements (3) bewegt wird, wodurch das Formelement von dem ersten Kopplungsmittel (17) gelöst wird.

8. Verfahren nach einem der Ansprüche 4 bis 7, wobei das erste röhrenförmige Element (3) aus der Form entnommen wird, indem das Formelement in einer kreisförmigen Bewegung um seine Längsachse bewegt wird, wodurch das Formelement von dem ersten Kopplungsmittel (17) gelöst wird.

## Revendications

1. Cathéter urinaire intermittent télescopique (1), comprenant un premier élément tubulaire (3) et un élément prolongateur (2) agencé de manière déplaçable dans une direction axiale à l'intérieur du premier élément tubulaire (3), entre au moins
- une première position axiale dans laquelle l'élément prolongateur (2) est déplaçable à l'intérieur du premier élément tubulaire (3), le premier élément tubulaire (3) comprenant un premier moyen de couplage (17) et l'élément prolongateur (2) comprenant un deuxième moyen de couplage (19), et
- une deuxième position axiale dans laquelle les premier et deuxième moyens de couplage (17, 19) coopèrent l'un avec l'autre pour limiter le déplacement dudit élément prolongateur (2) dans au moins une direction longitudinale,
- le premier et/ou le deuxième moyen de couplage (17, 19) étant munis d'au moins une saillie dans une direction radiale d'un axe longitudinal du premier élément tubulaire et/ou de l'élément prolongateur,
- **caractérisé en ce que** le premier moyen de couplage (17) est prévu sous forme de saillie hélicoïdale agencée sur la surface interne de l'élément tubulaire.

2. Cathéter urinaire intermittent télescopique (1) selon l'une quelconque des revendications précédentes, dans lequel le deuxième moyen de couplage (19) est agencé sur la surface extérieure de l'élément prolongateur.

3. Cathéter urinaire intermittent télescopique (1) selon l'une quelconque des revendications précédentes, dans lequel le premier moyen de couplage (17) est prévu à l'extrémité proximale du premier élément tubulaire et le deuxième moyen de couplage (19) est prévu à l'extrémité distale de l'élément prolongateur.

4. Procédé de fabrication d'un cathéter urinaire intermittent télescopique (1) selon les revendications 1 à 3, comprenant les étapes consistant à
- prévoir un élément prolongateur (2) présentant au moins une pointe de cathéter insérable, un corps de cathéter présentant un conduit interne et un deuxième moyen de couplage (19),
- injecter une matière de cathéter fluide dans un moule formé pour définir un premier élément tubulaire (3) présentant un conduit interne d'une taille permettant à l'élément prolongateur (2) d'être positionné de manière déplaçable à l'intérieur du premier élément tubulaire (3),
- le premier élément tubulaire (3) étant muni d'un premier moyen de couplage moulé (17) destiné à coopérer avec le deuxième moyen de couplage (19) de l'élément prolongateur, et
- solidifier la matière à l'intérieur de celui-ci,
- les premiers moyens de couplage étant moulés pour définir une saillie hélicoïdale (17) dans une direction radiale sur la surface interne du premier élément tubulaire.

5. Procédé selon la revendication 4, dans lequel le premier élément tubulaire (3) est moulé autour d'un élément de moulage définissant le conduit interne du premier élément tubulaire et du premier moyen de couplage (17).

6. Procédé selon l'une quelconque des revendications 4 à 5, dans lequel le premier élément tubulaire (3) est retiré du moule sans déformer la matière moulée pendant le retrait.

7. Procédé selon l'une quelconque des revendications 4 à 6, dans lequel le premier élément tubulaire (3) est retiré du moule en manœuvrant le premier élément tubulaire (3) dans un mouvement circulaire autour de l'axe longitudinal du premier élément tubulaire (3) libérant l'élément de moulage du premier moyen de couplage (17).

8. Procédé selon l'une quelconque des revendications 4 à 7, dans lequel le premier élément tubulaire (3) est retiré du moule en manœuvrant l'élément de moulage dans un mouvement circulaire autour de son axe longitudinal libérant l'élément de moulage du premier moyen de couplage (17).
